# FASCICULE DE BREVET EUROPEEN

(11) **EP 1 110 931 B1**
(45) Date de publication et mention de la délivrance du brevet: **09.07.2003**
(21) Numéro de dépôt: 00403413.8
(22) Date de dépôt: 06.12.2000
(51) Int. Cl.: C07C 2/54, C07C 5/27

(54) **Procédé et dispositif pour l'alkylation de l'isobutane par des oléfines légères.**
Verfahren und Vorrichtung zur Alkylierung von Isobutan mittels leichten Olefinen
Process and device for the alkylation of isobutane with light olefins

(30) Priorité: 23.12.1999 FR 9916375
(43) Date de publication de la demande: 27.06.2001
(73) Titulaire: TotalFinaElf France, 92800 Puteaux (FR)
(72) Inventeur: Mauleon, Jean-Louis, 27500 Sainte Croix Aizier (FR); Nascimento, Pedro, 76620 Le Havre (FR)
(74) Mandataire: Jolly, Jean-Pierre

(56) Documents cités:
- US-A- 2 394 906
- US-A- 4 324 937

## Description

La présente invention constitue une amélioration aux procédés d'alkylation de l'isobutane par des oléfines légères. Plus précisément, la présente invention concerne un procédé d'alkylation de l'isobutane par des hydrocarbures oléfiniques, dans lequel on extrait des effluents réactionnels une coupe riche en normal-butane, que l'on valorise par isomérisation, de manière à produire de l'isobutane destiné à être recyclé en charge de l'unité d'alkylation.

L'invention concerne également un dispositif pour la mise en oeuvre d'un tel procédé.

Les raffineurs ont développé, à partir des années 1930, des procédés d'alkylation, dans lesquels des paraffines ramifiées (ou isoparaffines) réagissent avec des oléfines pour former des hydrocarbures supérieurs ramifiés, essentiellement destinés à être incorporés dans les essences pour avions, ou à formuler des essences automobiles à haut indice d'octane.

Les conditions industrielles de mise en oeuvre de la réaction d'alkylation de l'isobutane par des oléfines légères sont bien connues de l'homme du métier. On pourra à cet effet se référer à l'article de J.F. Joly, dans l'ouvrage intitulé Procédés de Transformation (Le Raffinage du Pétrole, Vol.3, Chap. 7 ; Edition Technip, 1998). De manière générale, deux charges, l'une contenant de l'isobutane et l'autre contenant des oléfines légères, sont mises en contact dans un réacteur d'alkylation, en présence d'un catalyseur de type acide, liquide ou solide. Les effluents réactionnels sont alors traités dans une zone de fractionnement, dans laquelle on sépare par distillation le produit recherché, ou alkylat, sous forme d'un mélange d'hydrocarbures comportant pour la plupart 5 à 12 atomes de carbone.

De manière connue en soi, les quantités relatives d'isobutane et d'oléfines introduites en charge de l'unité d'alkylation constituent un paramètre clef dans la mise en oeuvre du procédé. En effet, le rapport isobutane/oléfines conditionne non seulement le rendement de la réaction, mais également la qualité de l'alkylat produit. On sait qu'il est indispensable d'utiliser un large excès d'isobutane par rapport aux oléfines, afin de limiter les réactions secondaires de polymérisation des oléfines. C'est pourquoi il est usuel d'opérer, dans les unités d'alkylation industrielles, avec un rapport molaire isobutane/oléfines compris entre 3 et 15 selon le type d'unité.

Il est donc indispensable de disposer d'une source importante d'isobutane pour alimenter l'unité d'alkylation. Or, l'isobutane est généralement produit en quantité limitée dans les raffineries, à moins de construire, en amont de l'unité d'alkylation, une unité telle que celles décrites dans l'article de G.L.Frischkorn, P.J.Kuchar et R.K.Olson, Energy Progress, 8(3), 154, 1988, et qui sont spécifiquement destinées à la production d'isobutane par isomérisation du normal-butane, mais qui représentent un investissement coûteux.

La quantité d'isobutane disponible limite donc souvent les capacités des unités industrielles d'alkylation. Dès lors, on comprend qu'il soit d'usage de récupérer, dans la colonne de fractionnement des effluents réactionnels, l'excès d'isobutane qui n'a pas réagi, et de le recycler en charge du réacteur d'alkylation.

Il est également d'usage courant de séparer, dans la colonne de fractionnement des effluents réactionnels, le normal-butane généralement présent dans ces effluents. En effet, la coupe riche en normal-butane ainsi obtenue est directement valorisable : elle est usuellement incorporée dans des produits tels que les essences ou les gaz de pétroles liquéfiés (GPL).

Une pratique particulièrement avantageuse consiste à faire subir à cette coupe riche en normal-butane un traitement d'isomérisation, qui permet de transformer le normal-butane en isobutane, que l'on peut alors recycler en charge de l'unité d'alkylation. Cette solution, mentionnée par exemple dans le brevet U.S. n° 5,675,052, est d'autant plus intéressante pour le raffineur qu'elle permet de valoriser davantage le normal-butane extrait des effluents, pour produire un appoint d'isobutane destiné à alimenter le procédé.

Cette solution apparaît également dans le brevet U.S. n° 5,565,617, qui décrit un système de fractionnement et de traitement spécifique adapté aux effluents issus d'un réacteur d'alkylation dans lequel la réaction se fait en présence d'un catalyseur acide solide et d'un composé halogéné. Ce traitement, destiné à éliminer les halogénures présents dans les effluents, incorpore également, en option, une étape de soutirage d'une coupe contenant du normal-butane, laquelle coupe est isomérisée avant d'être recyclée à l'entrée de la zone de fractionnement des effluents d'alkylation. Un tel agencement permet d'utiliser avantageusement la zone de fractionnement des effluents pour séparer le mélange de normal et d'isobutane sortant de la zone d'isomérisation.

Le brevet U.S. n° 4,324,937 décrit un procédé de production de coupes du type des essences à partir d'un mélange de propane et de butane. Ce procédé comporte une étape de production d'isobutane par isomérisation du normal-butane, une étape de production de propylène par déshydrogénation du propane et une étape de production d'hydrocarbures supérieurs par alkylation de l'isobutane par le propylène. La production d'isobutane est assurée par isomérisation tant du normal-butane présent dans le mélange de départ que de celui extrait des effluents de la réaction d'isomérisation. Optionnellement, la coupe riche en normal-butane peut être, avant isomérisation, fractionnée dans un déisobutaniseur dont la fonction est d'extraire de cette coupe l'isobutane résiduel, ce qui permet de déplacer l'équilibre de la réaction d'isomérisation du normal-butane en faveur de la production d'isobutane.

Toutefois, les systèmes proposés dans l'art antérieur se sont jusqu'à présent avérés peu satisfaisants. En particulier, les systèmes dans lesquels on isomérise, puis on recycle une coupe riche en normal-butane extraite des effluents réactionnels de l'alkylation, bien que potentiellement très avantageux, ne permettent pas de réaliser les gains escomptés.

En effet, si l'on utilise un seul réacteur d'isomérisation, le rendement de la conversion du normal-butane en isobutane est souvent insuffisant, et la quantité de l'appoint d'isobutane produit par ce système ne justifie pas l'investissement lié à l'installation et au fonctionnement du réacteur d'isomérisation.

Une solution consiste alors à utiliser deux réacteurs d'isomérisation en série, fonctionnant dans des conditions de sévérité différentes, comme cela est traditionnellement pratiqué dans les unités classiques d'isomérisation de normal-paraffines. Toutefois, cette alternative, si elle permet d'obtenir un rendement satisfaisant pour l'isomérisation du recycle de normal-butane, double l'investissement, puisqu'il faut alors construire et faire fonctionner deux réacteurs, si bien qu'un tel système s'avère in fine peu rentable.

Poursuivant ses recherches dans le domaine de l'alkylation, la Demanderesse s'est intéressée à ces systèmes dans lesquels on valorise par isomérisation une coupe riche en normal-butane extraite des effluents réactionnels, et elle a mis au point un procédé qui permet de remédier aux inconvénients de l'art antérieur.

En particulier, elle a découvert que, de manière surprenante, le fait d'extraire préalablement de ladite coupe riche en normal-butane les composés à 5 atomes de carbone et plus qui y sont présents, augmente considérablement le rendement de la réaction d'isomérisation du normal-butane.

La Demanderesse a en effet émis l'hypothèse selon laquelle les composés à au moins cinq atomes de carbone formés pendant la réaction d'alkylation, qui sont présents en quantité plus ou moins importante dans la coupe riche en normal-butane extraite des effluents réactionnels, avaient pour effet d'inhiber la réaction d'isomérisation du normal-butane, en se comportant comme des poisons de catalyseur. Elle a ainsi découvert que, en contrôlant rigoureusement la teneur de la coupe riche en normal-butane en composés à au moins cinq atomes de carbone (que l'on notera par la suite composés C₅⁺), on parvenait à augmenter notablement l'efficacité et donc la rentabilité du procédé.

La Demanderesse a ainsi mis au point un procédé d'alkylation de l'isobutane par des hydrocarbures oléfiniques, dans lequel on met en contact une première charge d'hydrocarbures riche en isobutane avec une seconde charge d'hydrocarbures riche en oléfines légères, dans des conditions propres à provoquer l'alkylation de l'isobutane par les oléfines légères, on traite les effluents issus de la zone réactionnelle dans une colonne de fractionnement afin d'en extraire au moins une première coupe riche en alkylat, une seconde coupe riche en normal-butane, et une troisième coupe riche en isobutane, puis on recycle ladite troisième coupe à l'entrée de la zone réactionnelle d'alkylation.

Ce procédé est caractérisé en ce que l'on applique à la seconde coupe (8) riche en normal-butane un traitement de purification en vue d'abaisser sa teneur en composés à 5 atomes de carbone et plus à une valeur inférieure ou égale à 5 % en poids, cette coupe ainsi purifiée étant ensuite traitée dans un réacteur d'isomérisation du normal-butane en isobutane, puis recyclée à l'entrée de la colonne de fractionnement des effluents d'alkylation.

Le procédé selon la présente invention permet de valoriser de manière optimale la coupe riche en normal-butane extraite des effluents réactionnels de l'alkylation, puisqu'il permet de produire, à partir de cette coupe, une quantité maximale d'isobutane destinée à réalimenter le réacteur d'alkylation. En effet, une fois débarrassée de la quasi-totalité des composés C₅⁺ qu'elle contient, cette coupe subit un traitement d'isomérisation, dont le rendement est d'autant plus élevé que ladite coupe contient moins de composés C₅⁺. Le mélange de normal- et d'isobutane ainsi obtenu est alors recyclé à l'entrée de la colonne utilisée pour le fractionnement des effluents réactionnels de l'alkylation. A cet effet, ce mélange peut être soit combiné, en amont de la colonne de fractionnement, aux effluents du réacteur d'alkylation, soit injecté directement dans ladite colonne de fractionnement des effluents réactionnels d'alkylation. Le mélange de normal et d'isobutane est ainsi fractionné en mélange avec les effluents réactionnels : l'isobutane qui a été produit dans le réacteur d'isomérisation est soutiré de la zone de fractionnement en mélange avec l'excès d'isobutane issu du réacteur d'alkylation, et le tout est recyclé en charge de l'unité d'alkylation. Quant au normal butane qui n'a pas été isomérisé dans le réacteur d'isomérisation, il est soutiré en mélange avec le normal butane présent dans les effluents réactionnels d'alkylation, et l'ensemble est dirigé vers le système d'extraction des composés C₅⁺ puis vers le réacteur d'isomérisation.

Un avantage majeur du procédé selon l'invention est donc qu'il permet d'augmenter substantiellement la quantité de troisième coupe riche en d'isobutane soutirée de la colonne de fractionnement des effluents réactionnels en vue du recyclage vers le réacteur d'alkylation. Ceci offre au raffineur la possibilité soit d'augmenter la capacité de production de l'unité d'alkylation, soit, à capacité de production constante, de diminuer l'apport d'isobutane frais en charge du réacteur d'alkylation. La rentabilité de l'unité s'en trouve ainsi sensiblement accrue.

Un autre avantage du procédé selon l'invention est qu'il rend superflue l'utilisation de deux réacteurs d'isomérisation en série pour la transformation en isobutane du normal-butane extrait des effluents réactionnels. En effet, une fois purifiée, c'est à dire débarrassée des composés C₅⁺, ladite seconde coupe riche en normal-butane peut être isomérisée avec un rendement satisfaisant dans un seul réacteur d'isomérisation. Les coûts de construction et de fonctionnement d'un système d'élimination des C₅⁺ étant nettement inférieurs à ceux d'un second réacteur d'isomérisation, le procédé selon l'invention offre une solution efficace, tout en limitant les investissements. Par rapport aux systèmes de l'art antérieur, le procédé selon l'invention permet donc de produire, à moindre coût et avec une efficacité optimale, un appoint non négligeable d'isobutane destiné à réalimenter le procédé d'alkylation.

La présente invention concerne également un dispositif permettant la mise en oeuvre du procédé décrit ci-avant.

L'invention a par conséquent pour objet un dispositif d'alkylation de l'isobutane par des hydrocarbures oléfiniques, comprenant au moins :
- une zone réactionnelle d'alkylation, de type connu en soi ;
- une colonne de fractionnement des effluents issus de ladite zone réactionnelle, comprenant au moins trois niveaux de soutirage : un niveau inférieur de soutirage d'une première coupe riche en alkylat, un niveau intermédiaire de soutirage d'une seconde coupe riche en normal-butane, et un niveau, supérieur au deux niveaux précédents, de soutirage d'une troisième coupe riche en isobutane ;
- un moyen de recyclage de ladite troisième coupe à l'entrée de la zone réactionnelle d'alkylation .

Ce dispositif se caractérise en ce qu'il comprend, disposés en série, au moins un moyen d'extraction sélective de ladite seconde coupe riche en normal-butane des hydrocarbures à 5 atomes de carbone et plus, un réacteur d'isomérisation du normal-butane en isobutane, et un moyen de recyclage de la seconde coupe isomérisée vers l'entrée de la colonne de fractionnement des effluents réactionnels d'alkylation.

Selon l'invention, on entend par moyen d'extraction sélective des hydrocarbures à 5 atomes de carbone et plus un système de purification apte à extraire sélectivement les composés C₅⁺ d'une coupe pétrolière, de manière suffisamment poussée pour réduire la teneur de ladite coupe en composés C₅⁺ à une valeur inférieure ou égale à 5% en poids, de préférence inférieure ou égale à 2% et, encore plus préférentiellement, inférieure ou égale à 1% en poids.

Le dispositif selon l'invention s'avère tout-à-fait approprié non seulement pour la construction de nouvelles unités d'alkylation, mais également dans le cadre de la modernisation d'unités traditionnelles, dans lesquelles il peut parfaitement être incorporé sans avoir à modifier les équipements déjà présents. Il suffit en effet d'ajouter un système d'extraction des C₅⁺, suivi d'un réacteur d'isomérisation, et de prévoir des moyens de recyclage de la seconde coupe vers l'entrée de la colonne de fractionnement des effluents. Le dispositif selon l'invention présente ainsi l'avantage d'être particulièrement simple à mettre en oeuvre, y compris dans les unités existantes.

Selon l'invention, il est essentiel d'extraire efficacement de ladite seconde coupe riche en normal-butane les composés C₅⁺ formés pendant la réaction d'alkylation, avant de diriger cette coupe vers le réacteur d'isomérisation. De préférence, on purifie cette coupe de manière à abaisser sa teneur en hydrocarbures à 5 atomes de carbone et plus à une valeur inférieure ou égale à 2% en poids et, encore plus préférentiellement, inférieure ou égale à 1% en poids.

Les moyens employés à cet effet peuvent être tous moyens permettant d'extraire d'une coupe pétrolière, de manière à la fois sélective et complète, les composés à au moins 5 atomes de carbone. Diverses techniques, bien connues de l'homme du métier, peuvent être mises en oeuvre.

De préférence, cette étape de purification est réalisée par distillation. Cette distillation est préférentiellement effectuée avec un point de coupe unique, qui peut être avantageusement choisi entre le point d'ébullition du normal-butane et le point d'ébullition de l'isopentane (ces deux points d'ébullition dépendant bien entendu de la pression à laquelle est réalisée la distillation). On purifie alors par distillation ladite seconde coupe riche en normal-butane, de manière à obtenir, d'une part, une coupe de tête constituant ladite seconde coupe purifiée et qui comprend le normal-butane et les composés plus légers que celui-ci et, d'autre part, une coupe de fond qui comprend les composés C₅⁺

Ledit moyen d'extraction des composés C₅⁺ comprend alors une colonne de distillation, qui peut être par exemple une colonne à plateaux ou à garnissage. Bien entendu, cette colonne doit comporter un nombre suffisant de plateaux, ou une hauteur suffisante de garnissage, pour permettre de réaliser la séparation des composés C₅⁺ de manière suffisamment complète afin d'en abaisser la teneur à la valeur requise.

De manière particulièrement avantageuse, ladite colonne de distillation est une colonne de rectification (également dénommée rectificateur), c'est-à-dire une colonne de distillation simplifiée comprenant uniquement une zone de rectification, mais pas de zone d'épuisement. Ce type de colonne présente l'avantage d'être particulièrement efficace pour le type de purification envisagé, tout en nécessitant un investissement modéré.

D'autres techniques de purification sont à la disposition de l'homme du métier et peuvent être employées à la place ou en complément de la distillation, telles que par exemple les séparations sur tamis moléculaires.

Les composés C₅⁺ extraits de ladite seconde coupe peuvent être par exemple réintroduits dans la colonne de fractionnement des effluents réactionnels d'alkylation, ou combinés à l'alkylat soutiré dans le fond de cette colonne.

L'étape d'isomérisation du normal-butane présent dans ladite seconde coupe purifiée est réalisée selon des méthodes classiques, avec la particularité que cette étape est préférentiellement réalisée dans un seul réacteur d'isomérisation. Ce réacteur renferme un catalyseur approprié, qui peut être tout catalyseur présentant une activité pour la réaction d'isomérisation du normal-butane. De tels catalyseurs, bien connus de l'homme du métier, sont généralement constitués de particules solides superacides.

En particulier, on peut avantageusement employer un catalyseur à base de zircone sulfatée associée à un métal du groupe VIII de la Classification Périodique des Eléments, tel que par exemple le catalyseur décrit dans la demande de brevet EP 908.232 au nom de la Demanderesse.

De manière encore plus avantageuse, on emploie un catalyseur comprenant, déposés sur un support tel que l'alumine, un métal du groupe VIII de la Classification Périodique des Eléments associé à des sites acides du type sites de Lewis, c'est à dire des halogénures métalliques de formule -OMX₂, dans laquelle X est un halogène, en particulier le chlore, et M est un métal, en particulier l'aluminium. Au moment de leur mise en oeuvre, il est préférable d'activer ces catalyseurs par transformation des sites de Lewis en sites de Brönsted de formule -OMX₃H, par halogénation par un agent halogénant tel que l'acide chlorhydrique, comme décrit par exemple dans les demandes de brevet WO 98/25699 et WO 97/19752.

Les conditions opératoires dans lesquelles s'effectuent l'isomérisation du normal-butane sont également connues. La coupe à isomériser est combinée à de l'hydrogène selon un rapport moléculaire hydrogène/hydrocarbures de préférence compris entre 0,005 et 10, puis circule, à une pression préférentiellement comprise entre 5.10⁵ et 50. 10⁵ Pa et à une température préférentiellement comprise entre 100 et 300°C, dans le réacteur d'isomérisation renfermant un ou plusieurs lits de catalyseur. Ces conditions opératoires sont avantageusement optimisées de manière à ce que l'intégralité du mélange réactionnel se trouve en phase vapeur dans le réacteur d'isomérisation.

A la sortie du réacteur d'isomérisation, la seconde coupe isomérisée est alors recyclée à l'entrée de la colonne de fractionnement des effluents réactionnels d'alkylation. Selon un mode de réalisation particulièrement avantageux, elle est directement réintroduite dans ladite colonne, à un niveau de préférence supérieur au niveau auquel s'effectue le soutirage de ladite seconde coupe riche en normal-butane. De manière encore plus préférée, elle est réintroduite à un niveau intermédiaire entre le niveau de soutirage de ladite seconde coupe riche en normal-butane et le niveau de soutirage de ladite troisième coupe riche en isobutane.

Deux charges d'hydrocarbures alimentent le procédé selon l'invention. La première charge est une coupe d'hydrocarbures riche en isobutane, comprenant de préférence au moins 70% en poids d'isobutane.

La seconde charge est une coupe d'hydrocarbures riche en oléfines légères, c'est à dire en mono-oléfines comprenant de 2 à 12 atomes de carbone. De préférence, ladite seconde charge renferme une quantité substantielle de mono-oléfines comprenant de 3 à 5 atomes de carbone : elle renferme avantageusement au moins 30% en poids de telles oléfines. Encore plus préférentiellement, ladite seconde charge contient au moins 40 % en poids de butènes.

Avantageusement, ladite seconde charge provient, en tout ou partie, des effluents d'une unité de craquage de coupes pétrolières lourdes telle qu'en particulier une unité de craquage catalytique. Une telle charge peut avantageusement, préalablement à son introduction dans le procédé selon l'invention, avoir subi un hydrotraitement visant à isomériser les mono-oléfines et/ou hydrogéner sélectivement les dioléfines présentes.

L'invention ne concerne pas les conditions dans lesquelles on effectue l'alkylation de l'isobutane par les oléfines légères. L'on peut en effet employer tout système connu, et l'homme du métier dispose à l'heure actuelle de diverses alternatives. La zone réactionnelle d'alkylation peut comprendre un ou plusieurs réacteurs, dans lesquels les deux charges sont mises en contact en présence d'un catalyseur généralement de type acide. Ce catalyseur peut être liquide (en particulier l'acide fluorhydrique ou l'acide sulfurique) ou solide (tel que par exemple le catalyseur décrit dans la demande de brevet WO/97.25141). Les conditions opératoires sont également connues de l'homme du métier. Elles incluent généralement une température peu élevée, de préférence comprise entre -10 et 150°C, et de préférence entre 0 et 100°C. La pression est avantageusement choisie à une valeur suffisamment élevée pour maintenir en phase liquide le mélange réactionnel au contact du catalyseur. Le rapport molaire isobutane/oléfines est avantageusement compris entre 3 et 15.

D'autres caractéristiques et avantages de l'invention apparaîtront à la lecture de la description de différents modes de réalisation, faite ci-après en référence aux dessins annexés , dans lesquels:
- la figure 1 est une vue schématique d'une unité d'alkylation conforme à l'invention ;
- la figure 2 est une vue plus détaillée d'un rectificateur de normal-butane, qui constitue un moyen préféré d'extraction des composés C₅⁺ de ladite seconde coupe riche en normal-butane.

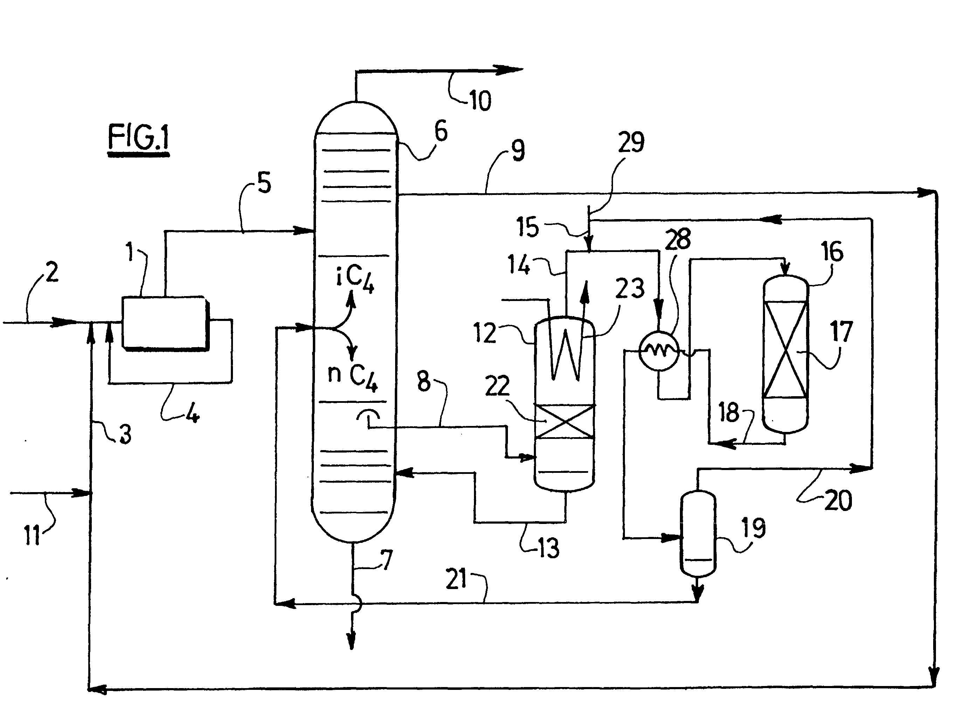

En référence à la figure 1, une première charge d'hydrocarbures 3 riche en isobutane et une seconde charge d'hydrocarbures 2 riche en oléfines légères alimentent un réacteur d'alkylation 1 fonctionnant en présence d'un catalyseur acide, par exemple liquide, tel que l'acide fluorhydrique.

A la sortie du réacteur, l'acide est récupéré pour être réintroduit dans l'unité par la ligne 4, et les effluents réactionnels sont dirigés par la ligne 5 vers la colonne de fractionnement 6, où ils sont séparés par distillation de manière à extraire :
- une première coupe riche en alkylat, soutirée en fond de la colonne 6 par la ligne 7 ;
- une seconde coupe riche en normal-butane, soutirée de la colonne 6 par la ligne 8 ;
- une troisième coupe riche en isobutane, soutirée de la colonne 6 par la ligne 9 ;
- une quatrième coupe, soutirée en tête de la colonne 6 par la ligne 10, qui comprend les composés légers : hydrocarbures à moins de quatre atomes de carbone, acide fluorhydrique résiduel, hydrogène,

La troisième coupe riche en isobutane soutirée par la ligne 9 est recyclée vers la zone réactionnelle d'alkylation : elle rejoint la ligne 3 d'alimentation du réacteur d'alkylation 1. Parallèlement, la ligne 11 permet d'acheminer vers la ligne d'alimentation 3 un appoint en isobutane frais, qui vient compléter l'isobutane recyclé.

La seconde coupe riche en normal-butane est acheminée par la ligne 8 vers l'enceinte 12, dans laquelle elle est purifiée par distillation, de manière à abaisser sa teneur en composés à cinq atomes de carbone et plus à une valeur inférieure ou égale à 5% en poids. Le flux des composés C₅⁺ extraits de ladite seconde coupe est soutiré de l'enceinte 12 par la ligne 13, et il est réinjecté dans la colonne 6 à un niveau de préférence inférieur au niveau de soutirage de ladite seconde coupe.

La seconde coupe purifiée, soutirée en tête de l'enceinte 12, est acheminée par la ligne 14 vers le réacteur d'isomérisation 16. Elle est mélangée à un gaz riche en hydrogène, amené par la ligne 15, et qui comprend de l'hydrogène de recycle amené par la ligne 20 et/ou un appoint d'hydrogène frais amené par la ligne 29. Le mélange résultant, après être passé dans l'échangeur de chaleur 28, est introduit dans le réacteur d'isomérisation 16, dans lequel il traverse un lit 17 de catalyseur d'isomérisation. Les effluents du réacteur 16 évacués par la ligne 18 passent dans l'échangeur de chaleur 28, avant d'être introduits dans le ballon le flash 19, dans lequel l'hydrogène résiduel est récupéré et recyclé en amont du réacteur d'isomérisation 16 par la ligne 20.

La seconde coupe ainsi isomérisée est alors recyclée par la ligne 21 vers la colonne 6 de fractionnement des effluents de l'alkylation, où elle est avantageusement injectée à un niveau intermédiaire entre le niveau (inférieur) de soutirage de ladite seconde coupe riche en normal-butane, et le niveau (supérieur) de soutirage de ladite troisième coupe riche en isobutane.

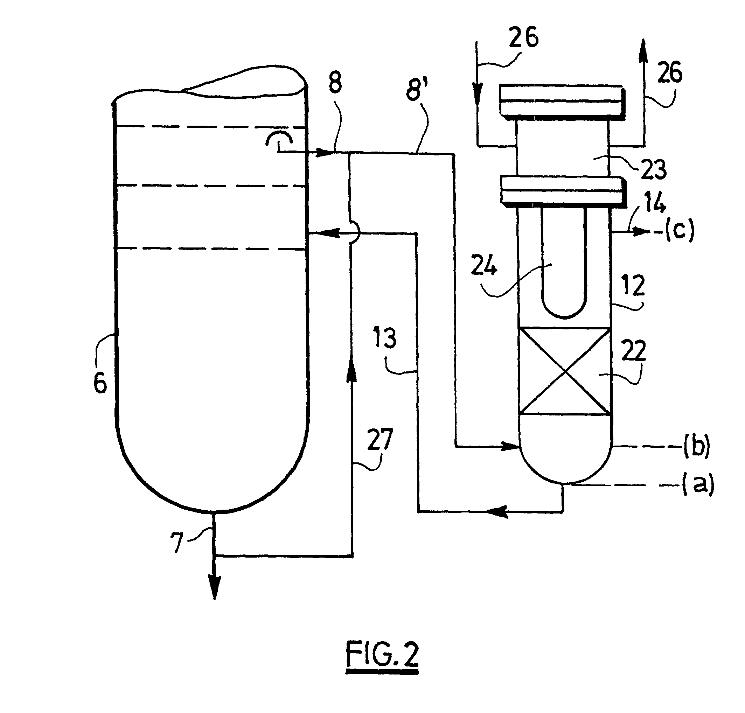

La figure 2 représente, de manière plus détaillée, un mode de réalisation préféré pour l'enceinte 12 de purification de la seconde coupe riche en normal-butane. Cette enceinte est constituée d'une colonne de distillation et, plus précisément, d'un rectificateur de normal-butane, qui permet d'abaisser la teneur de cette coupe en composés C₅⁺ à une valeur inférieure à 1% en poids. Cette colonne comprend deux niveaux de soutirage: un niveau supérieur (c) de soutirage, par la ligne 14, de ladite seconde coupe rectifiée (c'est-à-dire appauvrie en composés C₅⁺), et un niveau inférieur (a) de soutirage, par la ligne 15, d'une coupe comprenant les composés C₅⁺.

Ladite seconde coupe riche en normal-butane, extraite de la colonne 6 de fractionnement des effluents d'alkylation, est acheminée par les lignes successives 8 et 8' vers le rectificateur 12, où elle est introduite à un niveau (b) intermédiaire entre les deux niveaux de soutirage (a) et (c).

Entre le niveau (b) d'introduction de ladite seconde coupe et le niveau (c) de soutirage de cette coupe purifiée, s'étend une zone de rectification, comprenant un lit de garnissage 22. En tête du rectificateur 12, un condenseur 23 est alimenté en fluide de refroidissement, par exemple de l'eau, qui est amené par la ligne 25, circule dans l'échangeur 24 et est évacué par la ligne 26. Le système de condensation ici représenté est un système de condensation interne à la colonne de rectification 12. Bien entendu, d'autres systèmes, connus de l'homme du métier, peuvent être mis en oeuvre, tels que des systèmes de condensation externes à la colonne 12.

En option, la ligne 27 offre la possibilité de combiner une fraction de l'alkylat, soutiré de la colonne 6 par la ligne 7, avec ladite seconde coupe riche en normal-butane, soutirée de la colonne 6 par la ligne 8. Le mélange est alors acheminé vers le rectificateur 12 par la ligne 8'. Ceci permet d'améliorer l'étape de purification de ladite seconde coupe riche en normal-butane, en favorisant l'entraînement vers le fond du rectificateur des composés C₅⁺.

Les exemples ci-après, qui n'ont pas de caractère limitatif, sont uniquement destinés à illustrer la mise en oeuvre de l'invention et les avantages de celle-ci.

### EXEMPLES

### Exemple comparatif 1

Trois essais ont été effectués dans une unité d'alkylation expérimentale, comprenant un réacteur dans lequel une première charge riche en isobutane est mise en contact avec une seconde charge riche en oléfines, en présence d'un catalyseur acide liquide (acide fluorhydrique), à une température de 40°C et une pression de 15.10⁵ Pa.

Pour ces trois essais, on utilise une même charge riche en oléfines légères, dont le débit est de 25000 kg/h et la composition est la suivante :

| Hydrocarbures | % en poids |
|---|---|
| Propane | traces |
| Isobutane | 27,6 |
| Butènes | 59,8 |
| Normal-butane | 12,0 |
| C₅⁺ | 0,6 |

Le premier essai, E1, est effectué dans une unité conforme à celle représentée sur la figure 1, comprenant un rectificateur de normal-butane 12, mais pas de réacteur d'isomérisation du normal-butane 16. En conséquence, après purification dans le rectificateur 12, la seconde coupe riche en normal-butane est extraite de l'unité pour être par exemple vendue telle quelle.

Le tableau ci après illustre les résultats de ce test, en terme de composition en kg/h des flux d'hydrocarbures circulant dans différentes lignes de l'unité :

| | Ligne 11 (appoint d'isobutane) | Ligne 3 (isobutane total) | Ligne 9 (recycle d'isobutane) | Ligne 7 (alkylat) |
|---|---|---|---|---|
| Propane | 74 | 406 | 331 | 0 |
| Isobutane | 11748 | 142482 | 130734 | 2 |
| Butènes | 0 | 0 | 0 | 0 |
| Normal-butane | 597 | 16831 | 16234 | 504 |
| C₅⁺ | 0 | 3525 | 3525 | 30506 |
| Flux total d'hydrocarbures | 12419 | 163244 | 150824 | 31012 |

Le deuxième essai, E2, est effectué dans une unité conforme à celle représentée sur la figure 1, mais qui ne comprend pas le rectificateur de normal-butane 12 : la seconde coupe riche en normal-butane soutirée de la colonne de fractionnement 6 est directement introduite dans le réacteur 16 d'isomérisation du normal-butane. Des mesures ont montré que la teneur de cette coupe en composés C₅⁺ est de 23% en poids.

Le tableau ci après illustre les résultats de ce test , en terme de composition en kg/h des flux d'hydrocarbures dans les différentes lignes :

| | Ligne 11 (appoint d'isobutane) | Ligne 3 (isobutane total) | Ligne 9 (recycle d'isobutane) | Ligne 7 (alkylat) |
|---|---|---|---|---|
| Propane | 62 | 485 | 422 | 0 |
| Isobutane | 9838 | 142482 | 132644 | 20 |
| Butènes | 0 | 0 | 0 | 0 |
| Normal-butane | 500 | 21973 | 21473 | 1394 |
| C₅⁺ | 0 | 3637 | 3637 | 30530 |
| Flux total d'hydrocarbures | 10400 | 168577 | 158176 | 31944 |

Le troisième essai, E3, est réalisé conformément à l'invention, dans une unité conforme à celle représentée sur la figure 1. En particulier, ladite seconde coupe riche en normal-butane soutirée par la ligne 8 est purifiée dans le rectificateur 12, de manière à en abaisser la teneur en composés C₅⁺ à une valeur de 0,5% en poids. La coupe ainsi purifiée est alors traitée dans le réacteur 16 d'isomérisation du normal-butane, puis recyclée à l'entrée de la colonne 6 de fractionnement des effluents réactionnels de l'alkylation.

Le tableau ci après illustre les résultats de ce test , en terme de composition en kg/h des flux d'hydrocarbures dans les différentes lignes :

| | Ligne 11 (appoint d'isobutane) | Ligne 3 (isobutane total) | Ligne 9 (recycle d'isobutane) | Ligne 7 (alkylat) |
|---|---|---|---|---|
| Propane | P | 504 | 446 | 0 |
| Isobutane | 9193 | 142482 | 133289 | 2 |
| Butènes | 0 | 0 | 0 | 0 |
| Normal-butane | 467 | 22987 | 22521 | 699 |
| C₅⁺ | 0 | 3529 | 3529 | 30532 |
| Flux total d'hydrocarbures | 9718 | 169502 | 159785 | 31233 |

Les trois essais ci-dessus on été réalisés avec un flux d'isobutane total constant (142482 kg/h) introduit par la ligne 3 dans le réacteur d'alkylation. Le rendement en alkylat (flux C₅⁺ soutiré par la ligne 7) reste sensiblement constant (de l'ordre de 30530 kg/h). En d'autres termes, la quantité d'isobutane recyclé par la ligne 9 a été complétée en adaptant le débit de l'isobutane d'appoint amené par la ligne 11.

La comparaison des résultats des essais E1 et E2 permet de constater l'effet bénéfique de l'introduction d'un réacteur 16 effectuant l'isomérisation du normal-butane en isobutane que l'on recycle à l'entrée de la colonne de fractionnement 6. En effet, ceci permet de réduire substantiellement l'appoint d'isobutane frais introduit dans l'unité par la ligne 11 : de 11748 kg/h dans l'essai E1, il passe à 9838 kg/h dans l'essai E2, soit une diminution de 16,25% .

L'essai E3 permet de constater l'amélioration supplémentaire liée à la mise en oeuvre du procédé conforme à l'invention. En effet, l'appoint d'isobutane frais introduit dans l'unité par la ligne 11 est cette fois de 9193 kg/h, soit un gain de 21,75% par rapport à l'essai E1, et un gain de 6,55% par rapport à l'essai E2. L'invention permet ainsi une valorisation optimale de la seconde coupe riche en normal-butane extraite des effluents réactionnels : une purification appropriée de cette coupe suivie d'une isomérisation du normal-butane permettent de produire une quantité maximale d'isobutane destiné à réalimenter le réacteur d'alkylation. La rentabilité de l'unité s'en trouve sensiblement améliorée puisque, pour un même rendement en alkylat, la consommation en isobutane frais, produit relativement coûteux, est nettement diminuée.

### Exemple comparatif 2

Cet exemple, réalisé dans les conditions de l'essai E3 ci-avant, illustre l'influence du degré de purification, dans le rectificateur 12, de ladite seconde coupe riche en normal-butane.

Trois essais E4, E5 et E6, ont été réalisés, dans lesquels on a fait varier les conditions de fonctionnement du rectificateur de normal-butane, de manière à faire varier la teneur en composés C₅⁺ de ladite seconde coupe introduite par la ligne 14 dans le réacteur d'isomérisation 16.

Pour les trois essais, les conditions de fonctionnement du réacteur d'isomérisation sont identiques :
- température : 155°C,
- pression : 30.10⁵ Pa,
- rapport moléculaire hydrogène / hydrocarbures : 0,5,
- pph (poids de charge par unité de poids de catalyseur et par heure) : 1 h⁻¹ ;
- catalyseur : à base de chlorure d'aluminium et de platine déposés sur un support d'alumine.

Le tableau ci-dessous illustre les résultats obtenus, en terme de rendement de la réaction d'isomérisation du normal-butane en isobutane effectuée dans le réacteur (16), en fonction de la composition du flux introduit par la ligne (14) dans ce réacteur.

| Essai | Teneur en composés C4 (% en poids) | Teneur en composés C₅⁺ (% en poids) | Rendement de la réaction normal-butane → isobutane |
|---|---|---|---|
| E3 | 91 | 9 | 59% |
| E4 | 99,4 | 0,6 | 61% |
| E5 | 99,99 | 0,01 | 61,8% |

Cet essai illustre l'effet bénéfique d'une extraction poussée des composés C₅⁺ de la seconde coupe riche en normal-butane, préalablement à l'isomérisation de cette dernière. Pour les essais E4 et E5, réalisés conformément à l'invention, on obtient un meilleur rendement de la réaction d'isomérisation du normal-butane, par rapport à l'essai E3 où une quantité trop importante de C₅⁺ tend à inhiber la réaction. Ainsi, le fait de réduire suffisamment la teneur de ladite seconde coupe en composés C₅⁺ permet de produire plus d'isobutane de recycle, et donc d'améliorer les performances et la rentabilité de l'unité d'alkylation.

## Revendications

1. Procédé d'alkylation de l'isobutane par des hydrocarbures oléfiniques, dans lequel on met en contact une première charge d'hydrocarbures (3) riche en isobutane avec une seconde charge d'hydrocarbures riche en oléfines légères (2), dans des conditions propres à provoquer l'alkylation de l'isobutane par les oléfines légères, on traite les effluents issus de la zone réactionnelle dans une colonne de fractionnement (6) afin d'en extraire au moins une première coupe riche en alkylat (7), une seconde coupe riche en normal-butane (8), et une troisième coupe riche en isobutane (9), puis on recycle ladite troisième coupe (9) à l'entrée de la zone réactionnelle d'alkylation, **caractérisé en ce que** l'on applique à la seconde coupe (8) riche en normal-butane un traitement de purification en vue d'abaisser sa teneur en composés à 5 atomes de carbone et plus à une valeur inférieure ou égale à 5 % en poids, cette coupe (14) ainsi purifiée étant ensuite traitée dans un réacteur (16) d'isomérisation du normal-butane en isobutane, puis recyclée (en 21) à l'entrée de la colonne (6) de fractionnement des effluents d'alkylation.

2. Procédé selon la revendication 1, **caractérisé en ce que** ladite seconde coupe (8) riche en normal-butane est purifiée de manière à abaisser sa teneur en composés à 5 atomes de carbone et plus à une valeur inférieure ou égale à 2% en poids et, de préférence, inférieure ou égale à 1% en poids.

3. Procédé selon l'une quelconque des revendications précédentes, **caractérisé en ce que** l'étape d'isomérisation du normal-butane présent dans ladite seconde coupe purifiée (14) est réalisée dans un seul réacteur d'isomérisation (16).

4. Procédé selon l'une quelconque des revendications précédentes, **caractérisé en ce que** la seconde coupe isomérisée (21) est directement réintroduite dans la colonne (6) de fractionnement des effluents d'alkylation.

5. Procédé selon l'une quelconque des revendications précédentes, **caractérisé en ce que** la seconde coupe isomérisée (21) est injectée dans la colonne (6) de fractionnement des effluents d'alkylation, à un niveau supérieur au niveau auquel s'effectue le soutirage de ladite seconde coupe (8) riche en normal-butane.

6. Procédé selon la revendication 5, **caractérisé en ce que** la seconde coupe isomérisée (21) est injectée dans la colonne (6) de fractionnement des effluents d'alkylation, à un niveau intermédiaire entre le niveau de soutirage de ladite seconde coupe (8) riche en normal-butane et le niveau de soutirage de ladite troisième coupe (9) riche en isobutane.

7. Procédé selon l'une quelconque des revendications précédentes, **caractérisé en ce que** l'étape de purification de la seconde coupe (8) riche en normal-butane est réalisée par distillation (12).

8. Procédé selon la revendication précédente, **caractérisé en ce que** la distillation est réalisée avec un point de coupe unique, qui peut être avantageusement choisi entre le point d'ébullition du normal-butane et le point d'ébullition de l'iso-pentane.

9. Procédé selon l'une des revendications 7 ou 8, **caractérisé en ce que**, entre la colonne (6) de fractionnement des effluents d'alkylation et la colonne de distillation (12), une fraction de l'alkylat est combiné avec ladite seconde coupe riche en normal-butane.

10. Procédé selon l'une quelconque des revendications précédentes, **caractérisé en ce que** le catalyseur présent dans le réacteur d'isomérisation (16) est un catalyseur à base de zircone sulfatée associée à un métal du groupe VIII de la Classification Périodique des Eléments.

11. Procédé selon l'une quelconque des revendications précédentes, **caractérisé en ce que** le catalyseur présent dans le réacteur d'isomérisation (16) est un catalyseur comprenant, déposés sur un support tel que l'alumine, un métal du groupe VIII de la Classification Périodique des Eléments associé à des sites acides du type sites de Lewis, c'est à dire des halogénures métalliques de formule -OMX₂, dans laquelle X est un halogène, en particulier le chlore, et M est un métal, en particulier l'aluminium, et qui ont été transformés, au moment de la mise en oeuvre, en sites de Brônsted de formule -OMX₃H, par halogénation par un agent halogénant tel que l'acide chlorhydrique.

12. Procédé selon l'une quelconque des revendications précédentes, **caractérisé en ce que**, pour l'étape d'isomérisation du normal-butane, la coupe à isomériser est combinée à de l'hydrogène selon un rapport moléculaire hydrogène/hydrocarbures de préférence compris entre 0,005 et 10, puis circule, à une pression préférentiellement comprise entre 5.10⁵ et 50. 10⁵ Pa et à une température préférentiellement comprise entre 100 et 300°C, dans le réacteur d'isomérisation renfermant un ou plusieurs lits de catalyseur.

13. Dispositif d'alkylation de l'isobutane par des hydrocarbures oléfiniques, comprenant au moins :
- une zone réactionnelle d'alkylation (1), de type connu en soi,
- une colonne (6) de fractionnement des effluents issus de ladite zone réactionnelle (1), comprenant au moins trois niveaux de soutirage : un niveau inférieur de soutirage d'une première coupe (7) riche en alkylat, un niveau intermédiaire de soutirage d'une seconde coupe (8) riche en normal-butane, et un niveau, supérieur au deux niveaux précédents, de soutirage d'une troisième coupe (9) riche en isobutane,
- un moyen (9 ;3) de recyclage de ladite troisième coupe (9) à l'entrée de la zone réactionnelle d'alkylation (1),
**caractérisé en ce qu'**il comprend, disposés en série, au moins un moyen (12) d'extraction sélective de ladite seconde coupe (8) riche en normal-butane des hydrocarbures à 5 atomes de carbone et plus, un réacteur (16) d'isomérisation du normal-butane en isobutane, et un moyen (21) de recyclage de la seconde coupe isomérisée vers l'entrée de la colonne (6) de fractionnement des effluents réactionnels d'alkylation.

14. Dispositif selon la revendication 13, **caractérisé en ce que** ledit moyen (12) d'extraction sélective comprend une colonne de distillation, qui peut être notamment une colonne à plateaux ou à garnissage.

15. Dispositif selon la revendication 14, **caractérisé en ce que** la colonne de distillation est une colonne de rectification du normal-butane.

## Patentansprüche

1. Verfahren zur Alkylierung von Isobutan durch olefinische Kohlenwasserstoffe, wobei man eine erste Charge von Kohlenwasserstoffen (3), die reich an Isobutan ist, mit einer zweiten Charge von Kohlenwasserstoffen, die reich an leichten Olefinen ist (2), unter Bedingungen, die geeignet sind, die Alkylierung des Isobutans durch die leichten Olefine zu fördern, in Kontakt bringt; die aus der Reaktionszone austretenden Abströme in eine Fraktionierkolonne (6) leitet, um mindestens einen ersten, alkylatreichen Schnitt (7), einen zweiten, n-butanreichen Schnitt (8) und einen dritten, isobutanreichen Schnitt (9) zu extrahieren, worauf man den dritten Schnitt (9) in eine Reaktionszone für die Alkylierung zurückleitet, **dadurch gekennzeichnet, dass** man dem zweiten, n-butanreichen Schnitt (8) einer Reinigungsbehandlung unterzieht, um seinen Gehalt an Verbindungen mit 5 oder mehr Kohlenstoffatomen auf einen Wert von ≤ 5 Gew.-% zu reduzieren, diesen so gereinigten Schnitt (14) anschließend in einem Reaktor (16) zur Isomerisierung des n-Butans in Isobutan überführt und die Alkylierungs-Abströme dann (bei 21) am Eingang der Fraktionierkolonne (6) zurückleitet.

2. Verfahren nach Anspruch 1, **dadurch gekennzeichnet, dass** man den zweiten (n-butanreichen) Schnitt (8) reinigt, indem man seinen Gehalt an Verbindungen mit 5 oder mehr Kohlenstoffatomen auf einen Wert von ≤ 2 Gew.-%, vorzugsweise auf ≤ 1 Gew.-%, vermindert.

3. Verfahren nach einem der vorhergehenden Ansprüche, **dadurch gekennzeichnet, dass** man die Stufe der Isomerisierung des im zweiten gereinigten Schnitt (14) vorhandenen n-Butans in einem einzigen Isomerisierungsreaktor (16) durchführt.

4. Verfahren nach einem der vorhergehenden Ansprüche, **dadurch gekennzeichnet, dass** man den zweiten, isomerisierten Schnitt (21) direkt in die Fraktionierkolonne (6) für die Alkylierungs-Abströme zurückleitet.

5. Verfahren nach einem der vorhergehenden Ansprüche, **dadurch gekennzeichnet, dass** man den zweiten, isomerisierten Schnitt (21) in die Fraktionierkolonne (6) für die Abströme der Alkylierung einspritzt, und zwar auf einem Niveau, das über dem Niveau liegt, bei dem man die Entnahme des zweiten, n-butanreichen Schnitts (8) durchführt.

6. Verfahren nach Anspruch 5, **dadurch gekennzeichnet, dass** man den zweiten, isomerisierten Schnitt (21) in die Fraktionierkolonne (6) für die Abströme der Alkylierung einspritzt, und zwar auf einem Niveau, das zwischen dem Niveau der Entnahme des zweiten, n-butanreichen Schnitts (8) und dem Niveau der Entnahme des isobutanreichen dritten Schnitts liegt.

7. Verfahren nach einem der vorhergehenden Ansprüche, **dadurch gekennzeichnet, dass** man die Stufe der Reinigung des n-butanreichen zweiten Schnitts (8) durch Destillation (12) durchführt.

8. Verfahren nach dem vorhergehenden Anspruch, **dadurch gekennzeichnet, dass** man die Destillation bei einem einzigen Schnittpunkt durchführt, der vorteilhafterweise zwischen dem Siedepunkt des n-Butans und dem Siedepunkt des Isopentans liegt.

9. Verfahren nach einem der Ansprüche 7 oder 8, **dadurch gekennzeichnet, dass** man zwischen der Fraktionierkolonne (6) für die Alkylierungs-Abströme und der Destillationskolonne (12) eine Alkylatfraktion mit dem zweiten, n-butanreichen Schnitt kombiniert.

10. Verfahren nach einem der vorhergehenden Ansprüche, **dadurch gekennzeichnet, dass** der im Isomerisierungsreaktor (16) befindliche Katalysator einen Katalysator auf der Basis von Zirkonsulfat darstellt, der mit einem Metall der Gruppe VIII des Periodensystems der Elemente assoziiert ist.

11. Verfahren nach einem der vorhergehenden Ansprüche, **dadurch gekennzeichnet, dass** der im Isomerisierungsreaktor (16) befindliche Katalysator einen Katalysator darstellt, der auf einem Träger, wie Aluminiumoxid, ein Metall der Gruppe VIII des Periodensystems der Elemente, abgeschieden enthält, das mit sauren Zentren vom Lewis-Typ assoziiert ist, d.h. mit Metallhalogeniden der Formel -OMX₂, worin X ein Halogen, insbesondere Chlor, und M ein Metall, insbesondere Aluminium, darstellt, die im Zeitpunkt der Behandlung durch Halogenieren mit einem Halogenierungsmittel, wie Chlorwasserstoff, in Brönsted-Zentren der Formel -OMX₃H umgewandelt wurden.

12. Verfahren nach einem der vorhergehenden Ansprüche, **dadurch gekennzeichnet, dass** man für die Stufe der Isomerisierung des n-Butans den zu isomerisierenden Schnitt mit Wasserstoff kombiniert, und zwar in einem Molverhältnis Wasserstoff/Kohlenwasserstoffen von vorzugsweise 0,05 bis 10, worauf man ihn bei einem Druck von vorzugsweise 5 x 10⁵ bis 50 x 10⁵ Pa und einer Temperatur von vorzugsweise 100 bis 300°C, in den Isomerisierungsreaktor, der ein oder mehrere Katalysatorbetten enthält, zurückleitet.

13. Vorrichtung zur Alkylierung von Isobutan durch olefinische Kohlenwasserstoffe, enthaltend mindestens:
- eine Reaktionszone für die Alkylierung (1), eines an sich bekannten Typs,
- eine Fraktionierkolonne (6) für die Abströme aus der Reaktionszone (1), enthaltend mindestens drei Entnahmeniveaus, ein unteres Entnahmeniveau für einen ersten alkylatreichen Schnitt (7), ein Zwischen- Entnahmeniveau für einen zweiten, n-butanreichen Schnitt (8) und ein über den beiden genannten Niveaus angeordnetes Niveau zur Entnahme eines dritten, isobutanreichen Schnitts (10);
- eine Einrichtung (9; 3) zum Zurückleiten des dritten Schnitts (9) zum Eingang der Reaktionszone für die Alkylierung (1),
**dadurch gekennzeichnet, dass** sie in Reihe angeordnet, mindestens eine Einrichtung (12) zur selektiven Extraktion des zweiten, n-butanreichen Schnitts (8) von Kohlenwasserstoffen mit 5 oder mehr Kohlenstoffatomen, einen Reaktor (16) zur Isomerisierung von n-Butan zu Isobutan, und eine Einrichtung (21) zum Zurückleiten des zweiten, isomerisierten Schnitts zum Eingang der Fraktionierkolonne (6) für die Reaktionsabströme der Alkylierung aufweist.

14. Vorrichtung nach Anspruch 13, **dadurch gekennzeichnet, dass** die Einrichtung (12) zur selektiven Extraktion eine Destillierkolonne enthält, die insbesondere eine Plattenkolonne oder eine bestückte Kolonne sein kann.

15. Vorrichtung nach Anspruch 14, **dadurch gekennzeichnet, dass** die Destillierkolonne eine Rektifizierkolonne für n-Butan darstellt.

## Claims

1. A process for the alkylation of isobutane by olefinic hydrocarbons, in which a first hydrocarbon feedstock (3) rich in isobutane is brought into contact with a second hydrocarbon feedstock rich in light olefins (2), under conditions capable of causing alkylation of the isobutane by the light olefins, the effluents coming from the reaction zone are treated in a fractionating column (6) in order to extract at least a first cut rich in alkylate (7), a second cut rich in normal butane (8) and a third cut rich in isobutane (9), then said third cut (9) is recycled to the inlet of the alkylation reaction zone, **characterised in that** the second cut (8) rich in normal butane is subjected to a purification treatment with a view to lowering its content of compounds with 5 or more carbon atoms to a value lower than or equal to 5 % by weight, this cut (14) thus purified then being treated in a reactor (16) for isomerising the normal butane into isobutane, then recycled (at 21) to the inlet of the alkylation effluent fractionating column (6).

2. A process according to claim 1, **characterised in that** said second cut (8) rich in normal butane is purified in such a way as to lower its content of compounds with 5 or more carbon atoms to a value lower than or equal to 2 % by weight and, preferably, lower than or equal to 1 % by weight.

3. A process according to any one of the preceding claims, **characterised in that** the stage comprising isomerisation of the normal butane present in said purified second cut (14) is performed in a single isomerisation reactor (16).

4. A process according to any one of the preceding claims, **characterised in that** the isomerised second cut (21) is directly reintroduced into the alkylation effluent fractionating column (6).

5. A process according to any one of the preceding claims, **characterised in that** the isomerised second cut (21) is injected into the alkylation effluent fractionating column (6) at a level higher than the level at which said second cut (8) rich in normal butane is drawn off.

6. A process according to claim 5, **characterised in that** the isomerised second cut (21) is injected into the alkylation effluent fractionating column (6) at an intermediate level between the level at which said second cut (8) rich in normal butane is drawn off and the level at which said third cut (9) rich in isobutane is drawn off.

7. A process according to any one of the preceding claims, **characterised in that** the stage comprising purification of the second cut (8) rich in normal butane is performed by distillation (12).

8. A process according to the preceding claim, **characterised in that** distillation is performed with a single cut point, which may advantageously be selected between the boiling point of the normal butane and the boiling point of the isopentane.

9. A process according to one of claims 7 or 8, **characterised in that**, between the alkylation effluent fractionating column (6) and the distillation column (12), a fraction of the alkylate is combined with said second cut rich in normal butane.

10. A process according to any one of the preceding claims, **characterised in that** the catalyst present in the isomerisation reactor (16) is a sulfated zirconia-based catalyst associated with a metal from group VIII of the Periodic Table of Elements.

11. A process according to any one of the preceding claims, **characterised in that** the catalyst present in the isomerisation reactor (16) is a catalyst comprising, deposited on a support such as alumina, a metal from group VIII of the Periodic Table of Elements associated with acid sites of the Lewis site type, that is to say metal halides of the formula -OMX₂ in which X is a halogen, in particular chlorine, and M is a metal, in particular aluminium, which sites have been transformed, at the time of implementation, into Brønsted sites of the formula -OMX₃H by halogenation by a halogenating agent such as hydrochloric acid.

12. A process according to any one of the preceding claims, **characterised in that**, for the stage comprising isomerisation of the normal butane, the cut to be isomerised is combined with hydrogen in a hydrogen/hydrocarbon molar ratio preferably of between 0.005 and 10, then circulates, at a pressure preferably of between 5.10⁵ and 50.10⁵ Pa and at a temperature preferably of between 100 and 300°C, in the isomerisation reactor containing one or more catalyst beds.

13. A device for the alkylation of isobutane by olefinic hydrocarbons, comprising at least:
- one alkylation reaction zone (1), of a type known per se,
- one column (6) for fractionation of the effluents leaving said reaction zone (1), comprising at least three draw-off levels: a lower level at which a first cut (7) rich in alkylate is drawn off, an intermediate level at which a second cut (8) rich in normal butane is drawn off, and a level, higher than the previous two levels, at which a third cut (9) rich in isobutane is drawn off.
- a means (9; 3) for recycling said third cut (9) to the inlet of the alkylation reaction zone (1),
**characterised in that** it comprises, disposed in series, at least one means (12) for selective extraction from said second cut (8) rich in normal butane of the hydrocarbons with 5 or more carbon atoms, a reactor (16) for isomerising the normal butane into isobutane, and a means (21) for recycling the isomerised second cut to the inlet of the column (6) for fractionating the alkylation reaction effluents.

14. A device according to claim 13, **characterised in that** said selective extraction means (12) comprises a distillation column, which may be in particular a plate or packed column.

15. A device according to claim 14, **characterised in that** the distillation column is a normal butane rectification column.
